# EUROPEAN PATENT APPLICATION

(11) **EP 4 767 972 A1**
(43) Date of publication of application: **01.07.2026**
(21) Application number: 25227862.7
(22) Date of filing: 31.12.2025
(51) Int. Cl.: A61B 18/14, A61B 17/32

(54) **TECHNOLOGIES FOR CONTROLLING CLAMPING FORCE OF A SURGICAL INSTRUMENT DURING AN OPERATION MODE**

(30) Priority: 30.12.2024 US 202419004762
(71) Applicant: Cilag GmbH International, 6300 Zug (CH)
(72) Inventor: SCOGGINS, Patrick, Cincinnati, 45242 (US); DAASCH, Kyle, Cincinnati, 45242 (US)
(74) Representative: Carpmaels & Ransford LLP

(57) **Abstract**

A surgical instrument and associated method for controlling clamping force of a surgical instrument includes an end effector having a jaw assembly movable between an open state and a closed state, a trigger assembly operable to move the jaw assembly of the end effector between the open state and the closed state, and a clamping force augmentation system controllable to increase a clamping force of the jaw assembly of the end effector. A controller of the surgical instrument is configured to activate the clamping force augmentation system to increase the clamping force of the jaw assembly of the end effector in response to an RF mode of the surgical instrument being activated and (ii) the position sensor indicating that the present state of the jaw assembly is in the closed state.

## Description

### TECHNICAL FIELD

The present disclosure relates generally to energy-based surgical instruments and, more particularly, to surgical instruments and associated methods for controlling a clamping force of the surgical instrument.

### BACKGROUND

Energy-based surgical instruments are finding increasingly widespread applications in surgical procedures by virtue of their unique performance characteristics. Depending upon specific device configurations and operational parameters, energy-based surgical instruments can provide both transection of tissue and hemostasis of the tissue by coagulation, which may reduce or otherwise minimize patient trauma. Depending on the particular application, energy-based surgical instruments may utilize different surgical technologies including, for example, ultrasonic and/or electro-surgical (e.g., radio frequency (RF)) technologies.

A typical ultrasonic surgical instrument may include a handpiece containing an ultrasonic transducer and an elongated shaft assembly having a distally mounted end effector to effect the cutting and sealing of tissue. For example, the end effector may include a jaw assembly having an ultrasonic blade and a clamp arm, which may include a non-stick tissue pad or similar bed to receive the ultrasonic blade. In some cases, the elongated shaft assembly may be permanently affixed to the handpiece. In other cases, the elongated shaft assembly may be detachable from the handpiece, as in the case of a disposable shaft assembly or a shaft assembly that is interchangeable between different handpieces. In use, the end effector transmits ultrasonic energy to tissue brought into contact with the ultrasonic blade of the end effector to realize the cutting and sealing action. Such ultrasonic surgical devices may be configured for open surgical use, laparoscopic, and/or endoscopic surgical procedures including robotic-assisted procedures.

Ultrasonic energy cuts and coagulates tissue using temperatures lower than those used in electro-surgical procedures. Vibrating at high frequencies (e.g., 55,500 times per second), the ultrasonic blade denatures protein in the tissue to form a sticky coagulum. Pressure exerted on tissue by the ultrasonic blade surface collapses blood vessels and allows the coagulum to form a hemostatic seal. A surgeon can control the cutting speed and coagulation by the force applied to the tissue by the end effector, the time over which the force is applied, and the selected excursion level of the end effector.

In electro-surgical instruments, one or more electrodes are incorporated into the end effector and configured to apply therapeutic electrical current to the patient's tissue to create a hemostatic seal. In electro-surgical instruments that do not include a harmonic mode (i.e., do not include a harmonic blade), the end effector may be embodied as two clamp arms or jaws. In such embodiments, the electro-surgical instrument may include a separate mechanical knife or blade for cutting the tissue after the creation of the hemostatic seal, which may be incorporated into the elongated shaft attached to the end effector. In bi-polar embodiments, an active electrode have be attached to one of the clamp arms of the end effector and configured to introduce an electrical current into the tissue, which is received by a return electrode attached to the other clamp arm of the end effector (or as the blade itself in embodiments including a harmonic mode). Conversely, in mono-polar embodiments, the return electrode (e.g., a "grounding pad") may be separate from the electro-surgical instrument and located on a different part of the body of the patient. In some embodiments, the electro-surgical instrument may also be configured to apply a sub-therapeutic electrical current to the patient's tissue, which may be used for sensing purposes (e.g., measuring tissue impedance).

Electro-surgery forms hemostatic seals by generating heat in the tissue via the introduced electrical energy, which is embodied as radio frequency ("RF") energy. The particular frequency employed can vary based on the intended use of the electro-surgical instrument within the range of about 100kHz to 1 MHz, although higher frequencies can be employed in some embodiments. Additionally, sub-therapeutic frequencies may be used in some situations for purposes other than hemostatic sealing, such as performing various electrical measurements on the tissue.

It should be appreciated that some energy-based surgical instrument may employ dual or multi-modal technologies for the transection and/or hemostasis of patient tissue. For example, in some cases, an energy-based surgical instrument may include both ultrasonic and electro-surgical capabilities (e.g., by utilizing the ultrasonic blade as an electrode for the electro-surgery mode), which increases the surgical options provided by the surgical instrument to the surgeon.

### SUMMARY

According to an aspect of the present disclosure, a surgical instrument may include an end effector, a trigger assembly, a clamping force augmentation system, and a controller. The end effector may include a jaw assembly movable between an open state and a closed state and an ultrasonic blade. The trigger assembly may be operable to move the jaw assembly of the end effector between the open state and the closed state. Additionally, the trigger assembly may include a position sensor configured to produce sensor data indicative of a present state of the jaw assembly of the end effector. The clamping force augmentation system may be controllable to increase a clamping force of the jaw assembly of the end effector. The controller may be configured to control activation of an ultrasonic mode of the surgical system and a radio frequency (RF) mode of the surgical instrument. The controller may also be configured to activate the clamping force augmentation system to increase the clamping force of the jaw assembly of the end effector in response to (i) the RF mode being activated and (ii) the position sensor indicating that the present state of the jaw assembly is in the closed state.

In some embodiments, the trigger assembly may include a mode switch that is selectable to instruct the controller to activate the RF mode. Additionally, in some embodiments, to activate the clamping force augmentation system may include to engage a first clamping force gear of the clamping force augmentation system and a second clamping force gear of the clamping force augmentation to increase the clamping force of the jaw assembly of the end effector. In such embodiments, the trigger assembly may include a yoke configured to move along a yoke axis in response to operation of the trigger assembly to move the jaw assembly of the end effector between the open state and the closed state. Additionally, the clamping force augmentation system may include a clamping force motor including the first clamping force gear and wherein the yoke includes the second clamping force gear.

Additionally, in some embodiments, the yoke ma include a passageway having an opening defined on a proximal end of the yoke. In such embodiments, the second clamping force gear may be located within the passageway. Additionally, the first clamping force gear of the clamping force motor may be configured to be received within the passageway of the yoke to engage with the second clamping force gear when the clamping force augmentation system is activated. In such embodiments, the surgical system may further include a bias spring coupled to the yoke and the clamping force motor. The bias spring may be configured to bias the first clamping force gear away from the second clamping force gear along the yoke axis.

In some embodiments, the second clamping force gear may extend proximally away from a proximal end of the yoke. In such embodiments, the first clamping force gear may be configured to axially mesh with the second clamping force gear when the clamping force augmentation system is engaged. Additionally, in such embodiments, engagement of the first clamping force gear with the second clamping force gear may cause the yoke to move proximally along the yoke axis. Furthermore, in some embodiments, the controller may be configured to deactivate the clamping force augmentation system in response to (i) the ultrasonic mode being activated or (ii) the position sensor indicating that the present state of the jaw assembly is not in the closed state.

According to another aspect of the present disclosure, a method for controlling operation of a surgical instrument may include determining, by a controller of the surgical instrument, whether a jaw assembly of an end effector of the surgical instrument is in a closed state based on sensor data produced by a position sensor of a trigger assembly of the surgical instrument and determining, by the controller, whether a radio frequency (RF) mode of the surgical instrument has been activated. The method may also include activating, by the controller, a clamping force augmentation system of the surgical instrument to increase a clamping force of the jaw assembly of the end effector in response to a determination that (i) the jaw assembly is in the closed state and (ii) the RF mode has been activated.

In some embodiments, activating the clamping force augmentation system may include engaging a first clamping force gear of the clamping force augmentation system and a second clamping force gear of the clamping force augmentation to increase the clamping force of the jaw assembly of the end effector. In such embodiments, activating the clamping force augmentation may include inserting the first clamping force gear into a passageway defined in a proximal end of a yoke of a trigger assembly of the surgical instrument to engage the second clamping force gear located within the passageway. For example, engaging the first clamping force gear with the second clamping force gear may include causing the yoke to move proximally along a yoke axis.

In some embodiments, activating the clamping force augmentation may include engaging the first clamping force gear with the second clamping force gear that extends from a proximal end of a yoke of a trigger assembly of the surgical instrument. For example, engaging the first clamping force gear with the second clamping force gear may include axially meshing the first clamping force gear with the second clamping force gear. In some embodiments, the method may further include deactivating, by the controller, the clamping force augmentation system in response to (i) determining, by the controller, that the RF mode has been deactivated or (ii) determining, by the controller, that the jaw assembly of the end effector is not in the closed state.

According to a further aspect of the present disclosure, a surgical instrument may include an end effector, a trigger assembly, a clamping force augmentation system, and a controller. The end effector may include a jaw assembly movable between an open state and a closed state and an ultrasonic blade. The trigger assembly may be operable to move the jaw assembly of the end effector between the open state and the closed state. Additionally, the trigger assembly may include a position sensor configured to produce sensor data indicative of a present state of the jaw assembly of the end effector and a yoke configured to move along a yoke axis in response to operation of the trigger assembly to move the jaw assembly of the end effector between the open state and the closed state. The clamping force augmentation system may be controllable to increase a clamping force of the jaw assembly of the end effector. The clamping force augmentation system may include a first clamping force gear coupled to a clamping force motor and a second clamping force gear coupled to the yoke. The controller may be configured to control activation of a radio frequency (RF) mode of the surgical instrument. Additionally, the controller may be configured to activate the clamping force augmentation system to cause engagement of the first clamping force gear with the second clamping force gear in response to a determination that (i) the RF mode is activated and (ii) the jaw assembly is in the closed state.

In some embodiments, the yoke may include a passageway having an opening defined on a proximal end of the yoke. In such embodiments, the second clamping force gear may be located within the passageway. Additionally, in such embodiments, to cause engagement of the first clamping force gear with the second clamping force gear may include to receive the first clamping force gear within the passageway of the yoke.

### BRIEF DESCRIPTION OF THE DRAWINGS

The detailed description particularly refers to the following figures, in which:
FIG. 1 is a simplified diagram of an embodiment of a system for controlling clamping force of a surgical instrument;
FIG. 2 is a perspective view of an embodiment of the surgical instrument of the system of FIG. 1;
FIG. 3 is a side elevation view of a jaw assembly of an end effector of the surgical instrument of FIG. 2 in an open state;
FIG. 4 is a side elevation view of the jaw assembly of the end effector of the surgical instrument of FIG. 2 in a closed state;
FIG. 5 is an exploded view of a handle assembly of the surgical instrument of FIG. 2;
FIG. 6 is a block diagram of an embodiment of a clamping force augmentation system of the surgical instrument of FIG. 2 including a pair of clamping force gears;
FIG. 7 is a side elevation view of an embodiment of the clamping force augmentation system of FIG. 6 in which one of the clamping force gears is located within a passageway of a yoke of a trigger system of the surgical instrument of FIG. 2;
FIG. 8 is a side elevation view of the embodiment of the clamping force augmentation system of FIG. 6 with the clamping force augmentation system in a deactivated state;
FIG. 9 is a side elevation view of the embodiment of the clamping force augmentation system of FIG. 6 with the clamping force augmentation system in an activated state;
FIG. 10 is a side elevation view of another embodiment of the clamping force augmentation system of FIG. 6 in which one of the clamping force gears is attached to a proximal end of the yoke of the trigger system of the surgical instrument of FIG. 2 and shown in a deactivated state;
FIG. 11 is a side elevation view of the embodiment of the clamping force augmentation system of FIG. 10 with clamping force augmentation system in an activated state;
FIG. 12 is a simplified block diagram an embodiment of a control circuit of the surgical instrument of FIG. 2; and
FIGS. 13A and 13B is a simplified flow diagram of an embodiment of a method for controlling a clamping force of a surgical instrument, which may be executed by the control circuit of FIG. 12.

### DETAILED DESCRIPTION OF THE DRAWINGS

While the concepts of the present disclosure are susceptible to various modifications and alternative forms, specific illustrative embodiments thereof have been shown by way of example in the drawings and will herein be described in detail. It should be understood, however, that there is no intent to limit the concepts of the present disclosure to the particular forms disclosed, but on the contrary, the intention is to cover all modifications, equivalents, and alternatives falling within the spirit and scope of the invention as defined by the appended claims.

Terms representing anatomical references, such as anterior, posterior, medial, lateral, superior, inferior, etcetera, may be used throughout the specification in reference to the orthopaedic implants and surgical instruments described herein as well as in reference to the patient's natural anatomy. Such terms have well-understood meanings in both the study of anatomy and the field of orthopaedics. Use of such anatomical reference terms in the written description and claims is intended to be consistent with their well-understood meanings unless noted otherwise.

References in the specification to "one embodiment," "an embodiment," "an illustrative embodiment," etc., indicate that the embodiment described may include a particular feature, structure, or characteristic, but every embodiment may or may not necessarily include that particular feature, structure, or characteristic. Moreover, such phrases are not necessarily referring to the same embodiment. Further, when a particular feature, structure, or characteristic is described in connection with an embodiment, it is submitted that it is within the knowledge of one skilled in the art to effect such feature, structure, or characteristic in connection with other embodiments whether or not explicitly described. Additionally, it should be appreciated that items included in a list in the form of "at least one A, B, and C" can mean (A); (B); (C); (A and B); (A and C); (B and C); or (A, B, and C). Similarly, items listed in the form of "at least one of A, B, or C" can mean (A); (B); (C); (A and B); (A and C); (B and C); or (A, B, and C).

The disclosed embodiments may be implemented, in some cases, in hardware, firmware, software, or any combination thereof. The disclosed embodiments may also be implemented as instructions carried by or stored on a transitory or non-transitory machine-readable (e.g., computer-readable) storage medium, which may be read and executed by one or more processors. A machine-readable storage medium may be embodied as any storage device, mechanism, or other physical structure for storing or transmitting information in a form readable by a machine (e.g., a volatile or non-volatile memory, a media disc, or other media device).

In the drawings, some structural or method features may be shown in specific arrangements and/or orderings. However, it should be appreciated that such specific arrangements and/or orderings may not be required. Rather, in some embodiments, such features may be arranged in a different manner and/or order than shown in the illustrative figures. Additionally, the inclusion of a structural or method feature in a particular figure is not meant to imply that such feature is required in all embodiments and, in some embodiments, may not be included or may be combined with other features.

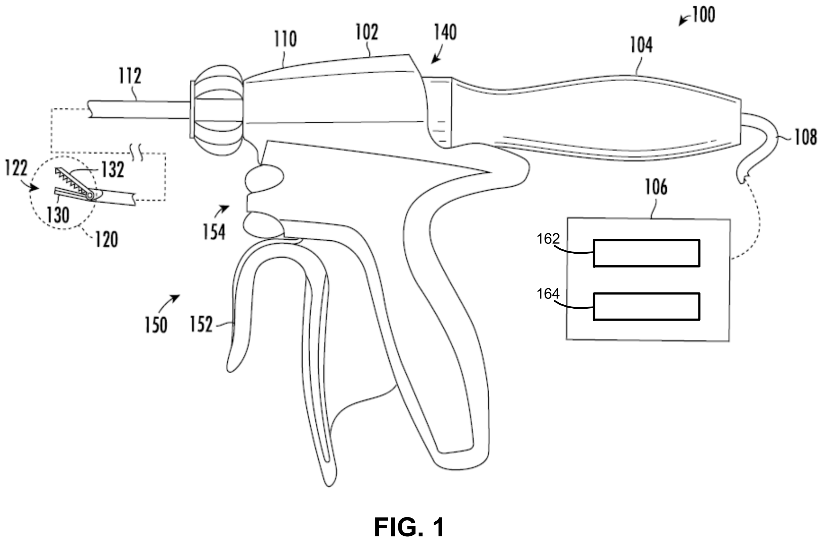

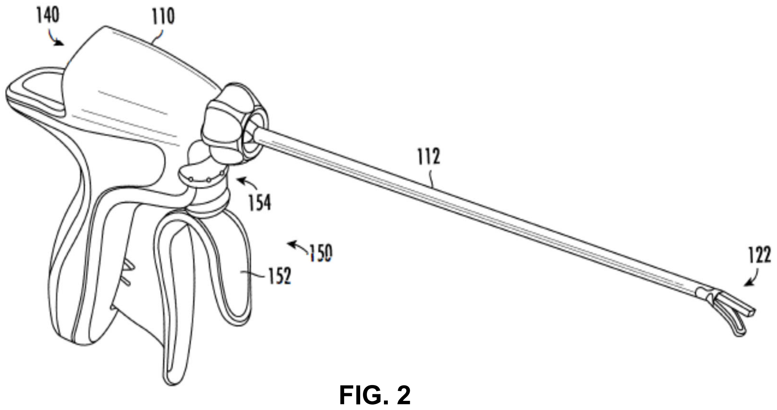

Referring now to FIGS. 1 and 2, in an illustrative embodiment, a system 100 for controlling a clamping force of a surgical instrument includes a multi-mode (e.g., ultrasonic and radio frequency (RF) modes) surgical instrument 102, a transducer 104, and a generator 106. In use, the surgical instrument 102 is usable to perform various surgical procedures including laparoscopic, endoscopic, or traditional open surgical procedures. In doing so, a surgeon may selectively activate various modes of the surgical instrument such as an ultrasonic mode and/or an RF mode. In the ultrasonic mode, the generator 106 drives the transducer 104 to cause an ultrasonic blade 130 of a jaw assembly 122 of an end effector 120 of the surgical instrument 102 to vibrate at a reference frequency, which facilitates the contemporaneous cutting and hemostatic sealing of patient tissue. In the RF mode, the generator 106 delivers an amount of therapeutic RF energy to the patient tissue to effect hemostatic sealing. In such embodiments, the blade 130 may be embodied as an ultrasonic blade 130 or as a mechanical blade designed to cut tissue using mechanical force (e.g., in those embodiments not employing ultrasonic technologies).

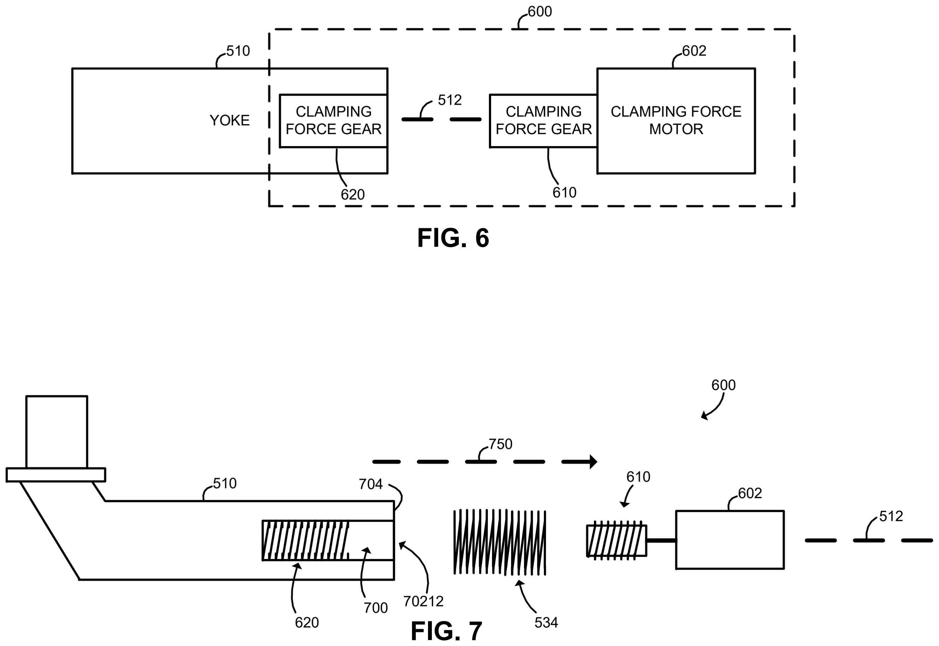

As discussed in more detail below, when the surgical instrument 102 is in the RF mode and the jaw assembly 122 is moved to the closed position, a clamping force augmentation system 600 (see FIG. 6) is activated to increase the clamping force of the jaw assembly 122 while in the RF mode. In this way, the clamping force of the jaw assembly 122 exerted on tissue captured with the jaw assembly 122 is increased while the surgical instrument is in the RF mode relative to the clamping force of the jaw assembly 122 while in the ultrasonic or other operation mode.

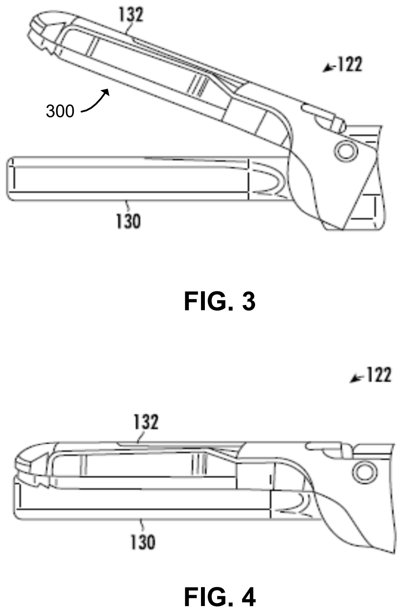

The surgical instrument 102 is illustratively embodied as surgical shears but may be embodied as other types of surgical instruments having a multiple modes of operation in other embodiments. In the illustrative embodiment, the surgical instrument 102 includes a handle assembly 110 and an elongated shaft assembly 112, which extends distally away from the handle assembly 110 and may be removably attached to the handle assembly 110 in some embodiments. The elongated shaft assembly 112 includes the end effector 120 located at a distal end away opposite the handle assembly 110. The end effector 120 includes the jaw assembly 122, which illustratively includes the ultrasonic blade 130 and a corresponding jaw clamp 132. As shown in FIGS. 3 and 4, the jaw assembly 122 is movable between an open state (FIG. 3) in which the jaw clamp 132 is positioned away from the ultrasonic blade 130 and a closed state (FIG. 4) in which the jaw clamp 132 is positioned near or otherwise contacts the ultrasonic blade 130. Actuation of the jaw assembly 122 from the open state to the closed state allows for the grasping, cutting, and coagulation of vessels and/or tissue by the jaw assembly 122. It should be appreciated that the open state may correspond to a degree of openness that is less than a fully opened position of the jaw assembly 122 and the closed state may correspond to a degree of closeness that is less than a fully closed position. That is, the open state may, for example correspond to a minimal distance or greater between the distal ends of the jaw clamp 132 and the ultrasonic blade 130 and the closed state may correspond to a maximum distance or less between the distal ends of the jaw clamp 132 and the ultrasonic blade 130. However, in other embodiments, the open state may correspond to a fully opened position of the jaw assembly 122 and the closed state may correspond to a fully closed position of the jaw assembly 122.

As indicated in FIG. 3, the jaw assembly 122 may include one or more electrodes 300 attached to or incorporated in the jaw clamp 132 and configured to deliver an amount of RF energy when the surgical instrument is in the RF mode. Although the illustrative end effector 120 includes only a single electrode 300 in the embodiment of FIG. 3, it should be appreciated that the end effector 120 may include additional electrodes 300 in other embodiments (e.g., multiple pads of electrodes 300). The electrode(s) 300 may be embodied as an active electrode configured to deliver the RF energy or as a return electrode configured to "sink" an applied RF energy. In those embodiments utilizing bi-polar RF implementation, the ultrasonic blade 130 may embody the active or return electrode, with the electrode 300 embodying the other active or return electrode. Alternatively, other active or return electrodes may be incorporated on the ultrasonic blade 130 or in another part of the jaw assembly 122 of the end effector 120. In mono-polar implementation, the RF electrode(s) 300 may be embodied as an active electrode, and a return electrode may be attached to a portion of the patient's body.

Referring back to FIGS. 1 and 2, the handle assembly 110 includes a receptacle 140 configured to receive the transducer 104 to facilitate connection of the transducer 104 to the handle assembly 110 and the elongated shaft assembly 112. The handle assembly 110 also includes a trigger assembly 150, which includes a primary trigger 152 and a switch assembly 154. The primary trigger 152 is operable by the surgeon to move the jaw assembly 122 of the end effector 120 between the open and closed states. The switch assembly 154 includes one or more buttons, which are selectable by the surgeon to activate (and configure, in some embodiments) the various modes of the surgical instrument 102, such as switching between the ultrasonic mode and the RF mode.

The transducer 104 is illustratively connected to the generator 106 by a cable assembly 108. As discussed above, the generator 106 is configured to drive the transducer 104 at a reference or resonate frequency to thereby cause the ultrasonic blade 130 to vibrate. For example, in an illustrative embodiment, the generator 106 may supply an electrical signal to the transducer 104 to cause the ultrasonic blade 130 of the jaw assembly 122 to vibrate longitudinally in the range of, for example, approximately 20 kHz to 250 kHz. In particular embodiments, for example, the ultrasonic blade 130 may vibrate in the range of about 54 kHz to 56 kHz (e.g., at about 55.5 kHz). In other embodiments, the ultrasonic blade 130 may vibrate at other frequencies including, for example, about 31 kHz or about 80 kHz. The excursion of the vibrations at the ultrasonic blade 130 can be controlled by, for example, controlling the amplitude of the electrical signal applied to the transducer 104 by the generator 106. The generator 106 may be activated so that electrical energy may be continuously or intermittently supplied to the transducer 104. The generator 106 also has a power line (not shown) for insertion in an electro-surgical unit or conventional electrical outlet. Additionally or alternatively, the generator 106 may be powered by a direct current (DC) source, such as a battery.

In the illustrative embodiment, the generator 106 may be configured to operate in different modes. As such, the illustrative generator 106 includes an ultrasonic generator module 162 for controlling an ultrasonic mode and an RF generator module 164 for controlling an RF or electro-surgical mode of the surgical instrument. The various modes of the generator 106 may be operated independently of each other in some embodiments. For example, the generator 106 may activate the ultrasonic mode of the ultrasonic generator module 162 to apply ultrasonic energy to the jaw assembly 122 and subsequently, either therapeutic or sub-therapeutic RF energy may be applied to the jaw assembly 122 by the electro-surgical generator module 164. Alternatively, the activation modes of the generator 106 may be operated simultaneously or contemporaneously with each other.

In the RF mode, the electro-surgical generator module 164 may be configured to generate RF energy at a frequency in the range of about 100 kilohertz (100 kHz) to about 1 megahertz (1 MHz). The generated RF energy is supplied to the patient's tissue via the electrodes 300 of the end effector 120 as described above in regard to FIG. 3. In some embodiments, the RF module 164 may also be configured to selectively provide the RF energy at sub-therapeutic levels to perform various electrical measurements of the patient's tissue. For example, the RF generator module 164 may be configured to measure an impedance of the patient's tissue using the electrodes 300 and a suitable RF energy level.

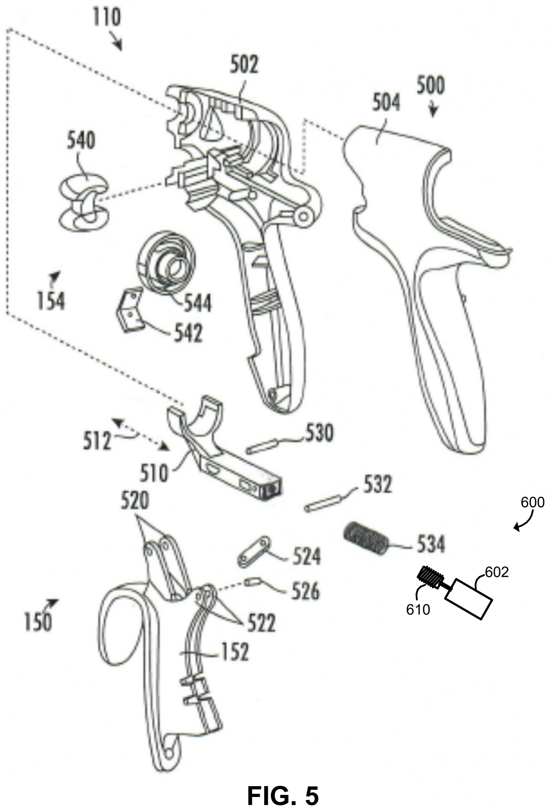

Referring now to FIG. 5, the handle assembly 110 illustratively includes a housing 500, which includes a right half-housing 502 and left half-housing 504. The half housings 502, 504 are configured to mate with each other to form the housing 500. To facilitate such mating, each of the half housings 502, 504 may include various interfaces sized to mechanically align and engage one another to form the housing 500 and enclose the internal working components of the surgical instrument 102.

The primary trigger 152 of the trigger assembly 150 is coupled to a linkage mechanism to translate the rotational motion of the primary trigger 152 to axial motion of a yoke 510 along a yoke axis 512, which in turn is configured to move the jaw assembly 122 of the end effector 120 between the open and closed states via the elongated shaft assembly 112. The primary trigger 152 includes a first set of flanges 520 having openings formed therein to receive a first yoke pin 530, which extends through the yoke 510 transverse to the yoke axis 512. The primary trigger 152 also includes a second set of flanges 522 configured to receive a first end of a link 524. A trigger pin 526 is received in openings formed in the first end of the link 524 and the second set of flanges 522. The trigger pin 526 forms a trigger pivot point for the primary trigger 152. A second end of the link 524, opposite the first end, is received in a slot formed in a proximal end of the yoke 510 and retained therein by a second yoke pin 532. As the primary trigger 152 is rotated about the pivot point formed from the trigger pin 526, the yoke 510 translates horizontally along the yoke axis 512. A spring 534 is used to bias the yoke 510 forward such that the jaw assembly 122 of the end effector 120 is biased to the open state (or a fully opened state).

As discussed above, the trigger assembly 150 also includes a switch assembly 154. The switch assembly 154 illustratively includes a toggle switch 540, which is selectable to activate one or more switches 542. Activation of the switches 542 electrically toggles or otherwise selects the various operation modes (e.g., ultrasonic or RF mode) of the surgical instrument.

The handle assembly 110 also includes the clamping force augmentation system 600. As shown in FIG. 6, the illustrative clamping fore augmentation system 600 includes a clamping force motor 602 having a clamping force gear 610 operatively attached thereto. For example, in the illustrative embodiment, the clamping force gear 610 is coupled to a shaft of the clamping force motor 602 so as to form a pinion gear of the clamping force motor 602. The clamping force augmentation system 600 also includes a clamping force gear 620 coupled to or otherwise incorporated in the yoke 510.

In use, as the trigger assembly 150 of the surgical instrument 102 is activated to move the jaw assembly 122 of the end effector 120 to the closed state, the yoke 510 is moved proximally (or "backwardly") along the yoke axis 512 such that the clamping force gear 620 of the yoke 510 is moved into proximity with the clamping force gear 610 of the clamping force motor 602. As such, when the clamping force augmentation system 600 is activated with jaw assembly 122 in the closed state, the clamping force gear 610 of the clamping force motor is engaged with clamping force gear 620 of the yoke 510. The engagement of the clamping force gears 610, 620 moves the yoke 510 further along the yoke axis 512 in the proximal direction, which further increases the clamping force exerted by the jaw assembly 122. It should be appreciated that the clamping force motor 602 may, alternatively or additionally, be moved toward the yoke 510 to cause engagement of the clamping for gears 610, 620 when the clamping force augmentation system 600 is activated.

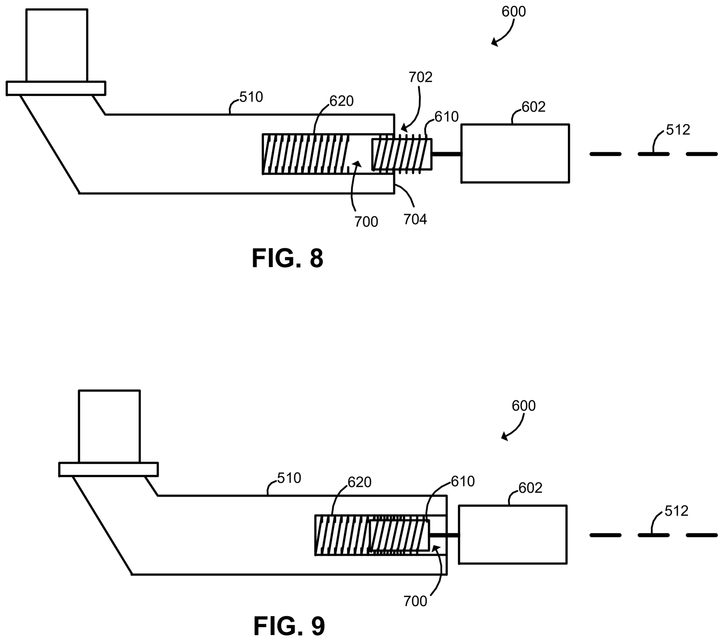

In an illustrative embodiment as shown in FIG. 7, the yoke 510 of the trigger assembly 150 includes a passageway 700 having an opening 702 defined in a proximal end 704 of the yoke 510. In such embodiments, the clamping force gear 620 is located within the passageway 700 as shown. For example, the clamping force gear 620 may be embodied as an internal gear defined within the internal walls of the passageway 700. As indicated in FIG. 7, as the trigger assembly 150 is activated to close the jaw assembly 122 of the end effector 120, the yoke 510 is moved in the proximal direction as indicated by arrow 750 along the yoke axis 512. As the yoke 510 is moved proximally along the yoke axis 512, the clamping force gear 610 of the clamping force motor 602 is received in the passageway 700 of the yoke 510 such that the clamping force gears 610, 620 are located in proximity to each other or otherwise initially contact each other as shown in FIG. 8. When the jaw assembly 122 is in the closed state and the clamping force augmentation system 600 is activated, the clamping force gear of the clamping force motor 602 further engages the clamping force gear 620 of the yoke 510 to cause the yoke to move further along the yoke axis 512 in the proximal direction indicated by arrow 750, which increases the clamping force of the jaw assembly 122. The resulting clamping force of the jaw assembly 122 can be controlled or otherwise modified by controlling the location of the yoke 510 along the yoke axis 512. As such, in some embodiments, the resulting clamping force is adjustable along a range of clamping forces.

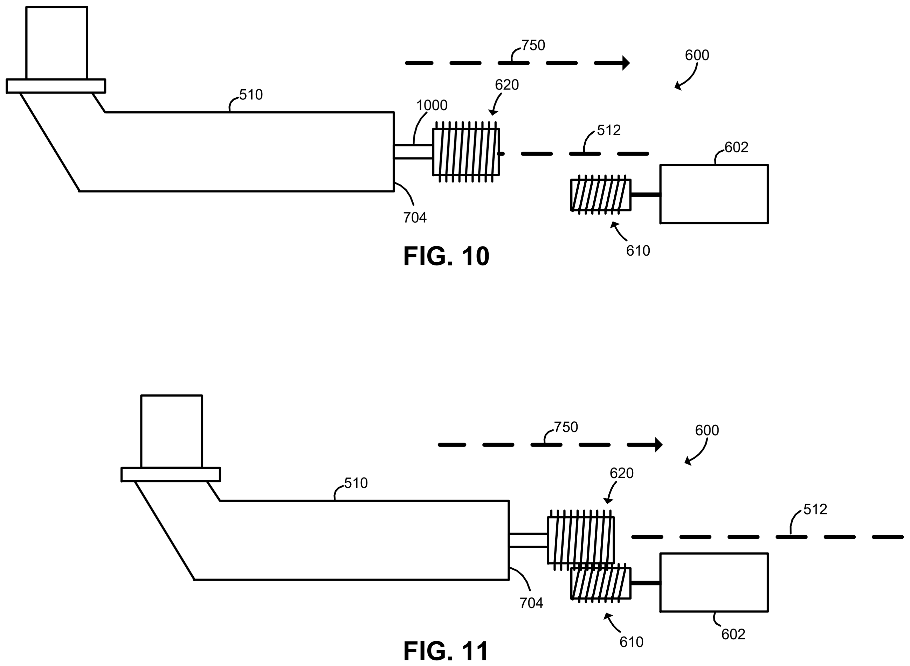

Referring now to FIGS. 10 and 11, in another embodiment, the clamping force gear 620 may be coupled to the proximal end 720. For example, as shown, the clamping force gear 620 may be attached to a non-rotating shaft 1000 extending from the proximal end 720 of the yoke 510. In such embodiments, as the as the trigger assembly 150 is activated to close the jaw assembly 122 of the end effector 120, the yoke 510 is moved in the proximal direction as indicated by arrow 750 along the yoke axis 512. As the yoke 510 is moved proximally along the yoke axis 512, the clamping force gear 620 of the yoke 510 is moved into proximity or otherwise into contact with the clamping force gear 610 of the clamping force motor 602. When the jaw assembly 122 is in the closed state and the clamping force augmentation system 600 is activated, the clamping force gear of the clamping force motor 602 further engages the clamping force gear 620 of the yoke 510 to cause the yoke to move further along the yoke axis 512 in the proximal direction indicated by arrow 750 as shown in FIG. 11, which increases the clamping force of the jaw assembly 122. Again, the resulting clamping force of the jaw assembly 122 can be controlled or otherwise modified by controlling the location of the yoke 510 along the yoke axis 512.

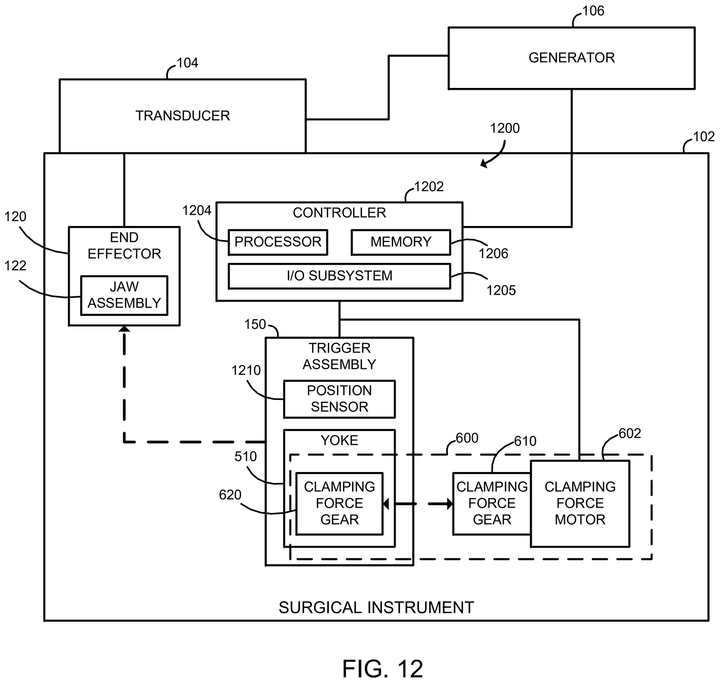

Referring now to FIG. 12, in the illustrative embodiment, the surgical instrument 102 includes a control circuit 1200. The control circuit 1200 includes a controller 1202, a position sensor 1210, and the clamping force augmentation system 600. In other embodiments, however, the control circuit 1200 may include additional or other electronic devices and/or circuit.

The controller 1202 may be embodied as any type of controller, functional block, digital logic, or other component, device, circuitry, or collection thereof capable of performing the functions described herein. In illustrative embodiment, the controller 1202 includes a processor 1204, a memory 1206, and an input/output (I/O) subsystem 1208. The processor 1204 may be embodied as any type of processor capable of performing the functions described herein. For example, the processor 1204 may be embodied as a single or multi-core processor(s), digital signal processor, microcontroller, or other processor or processing/controlling circuit. Similarly, the memory 1206 may be embodied as any type of volatile and/or non-volatile memory or data storage capable of performing the functions described herein. In operation, the memory 1206 may store various data and software used during operation of the control circuit 1200 such as executable firmware or software, programs, libraries, and drivers, which may be executed or otherwise used by the processor 1204.

The processor 1204 and memory 1206 are communicatively coupled to other components of the control circuit 1200 via the I/O subsystem 1208, which may be embodied as circuitry and/or components to facilitate input/output operations between the controller 1202 (e.g., the processor 1204 and the memory 1206) and the other components of the control circuit 1200. For example, the I/O subsystem 1208 may be embodied as, or otherwise include, memory controller hubs, input/output control hubs, firmware devices, communication links (i.e., point-to-point links, bus links, wires, cables, light guides, printed circuit board traces, etc.) and/or other components and subsystems to facilitate the input/output operations. In some embodiments, the I/O subsystem 1208 may form a portion of a system-on-a-chip (SoC) and be incorporated, along with the processor 1204 and the memory 1206, and other components of the surgical instrument 102, on a single integrated circuit chip. Additionally, in some embodiments, the memory 1206, or portions of the memory 1206, may be incorporated into the processor 1204.

The position sensor 1210 may be embodied as any type of sensor or sensing device or collection thereof capable of producing sensor data indicative of a present state of the jaw assembly 122 (e.g., whether the jaw assembly 122 is in the closed or open state). For example, in some embodiments, the position sensor 1210 may be embodied as a sensor coupled to the yoke 510 and configured to generate sensor data indicative of a present position of the yoke 510, which is indicative of the state of the jaw assembly 122 as discussed above. In such embodiments, the position sensor 1210 may be embodied as a hall effect sensor, a mechanical switch sensor, and/or other sensor capable to detecting the relative position of the yoke 510.

During operation, as discussed above, the controller 1202 is configured to control activation of the clamping force augmentation system 600 to thereby increase the clamping force of the jaw assembly 122 of the end effector 120. To do so, the controller 1202 is configured to monitor the sensor data produced by the position sensor 1210 to determine the present state of the jaw assembly 122. If the jaw assembly 122 is in the closed state and the RF mode of the surgical instrument is activated, the controller 1202 is configured to activate the clamping force augmentation system 600. As discussed above, when activated, the clamping force augmentation system 600 causes the yoke 510 to move further along the yoke axis in the proximal direction to thereby increase the resulting clamping force of the jaw assembly 122 on tissue captured by the jaw assembly 122. In doing so, the clamping force gears 610, 620 of the clamping force augmentation system 600 are engaged with each other to control the movement of the yoke 510 as discussed above.

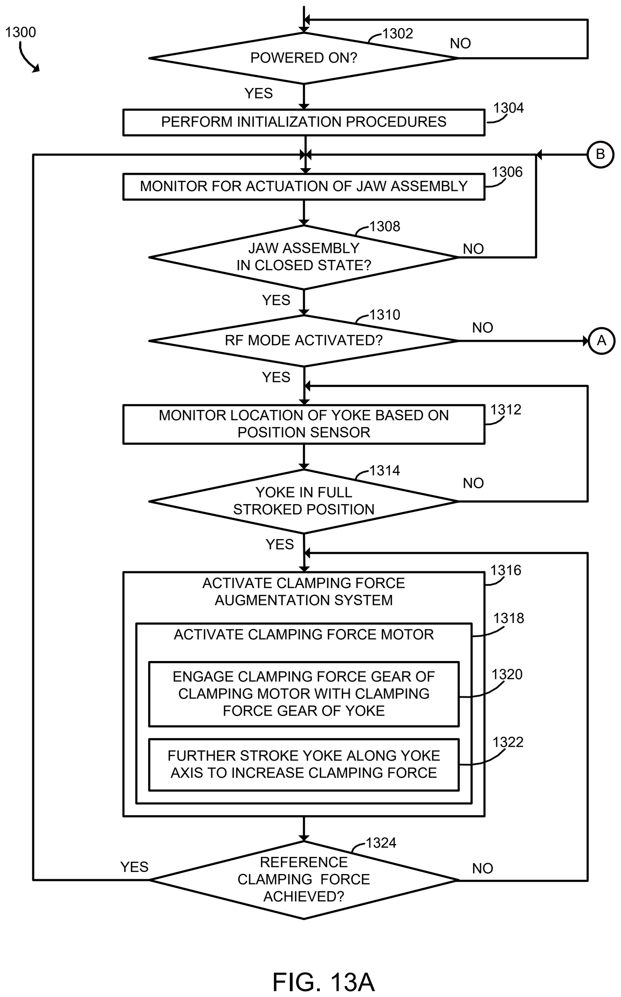

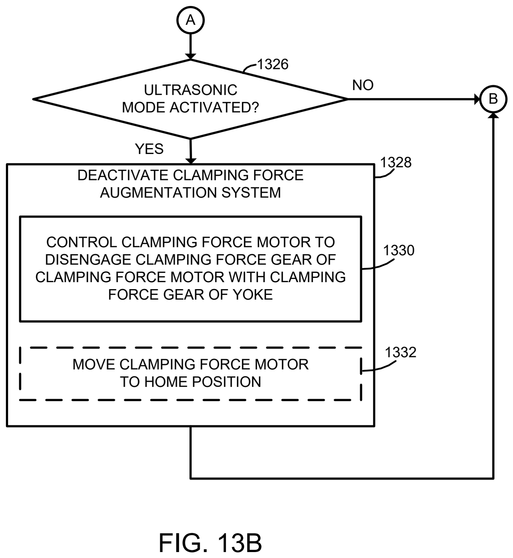

Referring now to FIGS. 13A & 13B, in use, the controller 1202 of the surgical instrument 102 may be configured to execute a method 1300 for controlling the clamping force of the jaw assembly 122 of the end effector 120 during an RF mode of the surgical instrument. The method 1300 begins with block 1302 in which controller 1202 performs one or more initialization procedures. For example, the controller 1202 may verify operation of the position sensor 1210, the clamping force augmentation system 600, and/or other initialization or verification procedure.

After the controller 1202 has performed the initialization procedures in block 1304, the method 1300 advances to block 1306 in which the controller 1202 monitors for actuation of the jaw assembly 122. To do so, the controller 1202 may monitor position data produced by the position sensor 1210, which is indicative of the position of the yoke 510 and, as such, activation or movement of the jaw assembly 122. In block 1308, the controller 1202 determines whether the jaw assembly 122 has been moved to the closed state based on the position data received from the position sensor 1210. Again, to do so, the controller 1202 may determine whether the yoke 510 is located in a position, as indicated by the position data form the position sensor 1210, that correlates to a closed position of the jaw assembly 122. If the jaw assembly is not in the closed position, the method 1300 loops back to block 1306 in which the controller 1202 continues monitoring monitors for actuation of the jaw assembly 122.

If, however, the controller 1202 determines that the jaw assembly 122 is in the closed state, the method 1300 advances to block 1310 in which the controller 1202 determines whether the RF mode of the surgical instrument 102 has been activated. If so, the method 1300 advances to block 1312 in which the controller 1202 further monitors the location of the yoke 510, as indicated by the sensor data from the position sensor 1210, and determines whether the yoke 510 is in a fully stroked position in block 1314. When the yoke 510 is in the fully stroked position, the jaw assembly is in a fully closed state and the clamping force gears 610, 620 are in proximity to or in contact with each other, as described above. For example, in the embodiments shown in FIG. 7-9, the clamping force gear 610 of the clamping force motor 602 may be received in the passageway 700 of the yoke 510 such that clamping force gears 610, 620 are in contact with each other when the yoke 510 is in the fully stroked position. If the yoke 510 is not in the fully stroked position, the method 1300 loops back to block 1312 in which the controller 1202 continues to monitor the location of the yoke 510.

If, however, the controller 1202 determines that the yoke 510 has been moved to a fully stoked positioned, the method 1300 advances to block 1316. In block 1316, the controller 1202 activates the clamping force augmentation system 600. In doing so, in block 1318, the controller 1202 activates and controls the operation of the clamping force motor 602 in block 1318. The activation of the clamping force motor 602 causes the clamping force gear 610 of the clamping force motor 602 to engage or mesh with the clamping force gear 620 of the yoke 510 in block 1320. The engagement of the clamping force gears 610, 620 and the further activation and control of the clamping force motor 602 further strokes or moves the yoke 510 along the yoke axis 512 in the proximal direction as indicated by arrow 750 in FIG. 7. As discussed above, the further movement or stroking of the yoke 510 along the yoke axis 512 increases the clamping force exerted by the jaw assembly 122.

Subsequently, in block 1324, the controller 1202 determines whether a reference clamping force has been achieved. The controller 1202 may determine the present clamping force based on the present position of the yoke 510 and/or based upon sensor data from a force sensor or the like. Additionally, the controller 1202 may adjust the reference clamping force based on various criteria, such as the type or model of the surgical instrument, the particular surgical procedure being performed, historical preference data, the present position of the trigger assembly 150, and/or other criteria.

If the controller 1202 determines that the reference clamping force has not been achieved, the method 1300 loops back to block 1316 in which the controller 1202 continues to activate the clamping force augmentation system 600 to obtain the desired or reference clamping force of the jaw assembly 122. If, however, the reference clamping force has been achieved, the method 1300 loops back to block 1306 in which the controller continues to monitor for actuation of the jaw assembly 122 as discussed above.

Referring back to block 1310, if the controller 1302 determines that the jaw assembly is in a closed state in block 1308 but the RF mode has not bee activated in block 1310, the method 1300 advances to bock 1326 of FIG. 13B. In block 1326, the controller 1202 determines whether an ultrasonic mode of the surgical instrument 102 has been activated. If not, the method 1300 loops back to block 1306 of FIG. 13A in which the controller 1202 continues to monitor for actuation of the jaw assembly 122. If, however, the controller 1202 determines that the ultrasonic mode has been activated, the method 1300 advances to block 1328. In block 1328, the controller 1202 deactivates the clamping force augmentation system 600. To do so, in block 1330, the controller 1202 controls the clamping force motor 602 to disengage the clamping force gear 610 of the clamping force motor 602 from the clamping force gear 620 of the yoke 510. Additionally, in embodiments in which the clamping force motor 602 is moved during activation of the clamping force augmentation system 600, the clamping force motor 602 is moved back to a home position in block 1332. The method 1300 subsequently loops back to block 1306 of FIG. 13A in which the controller 1202 continues to monitor for actuation of the jaw assembly 122 as discussed above. In this way, the controller 1202 is configured to control augmentation of the clamping force of the jaw assembly 122 by selectively engaging and disengaging the clamping force gears 610, 620 from each other.

The following is a non-exhaustive list of embodiments of the invention which may or may not be claimed:
1. A surgical instrument comprising:
   an end effector having a jaw assembly movable between an open state and a closed state, wherein the jaw assembly includes an ultrasonic blade;
   a trigger assembly operable to move the jaw assembly of the end effector between the open state and the closed state, wherein the trigger assembly includes a position sensor configured to produce sensor data indicative of a present state of the jaw assembly of the end effector;
   a clamping force augmentation system controllable to increase a clamping force of the jaw assembly of the end effector;
   a controller configured to control activation of an ultrasonic mode of the surgical system and a radio frequency (RF) mode of the surgical instrument, wherein the controller is configured to activate the clamping force augmentation system to increase the clamping force of the jaw assembly of the end effector in response to (i) the RF mode being activated and (ii) the position sensor indicating that the present state of the jaw assembly is in the closed state.
2. The surgical system of embodiment 1, wherein the trigger assembly includes a mode switch that is selectable to instruct the controller to activate the RF mode.
3. The surgical system of embodiment 1, wherein to activate the clamping force augmentation system comprises to engage a first clamping force gear of the clamping force augmentation system and a second clamping force gear of the clamping force augmentation to increase the clamping force of the jaw assembly of the end effector.
4. The surgical system of embodiment 3, wherein the trigger assembly includes a yoke configured to move along a yoke axis in response to operation of the trigger assembly to move the jaw assembly of the end effector between the open state and the closed state, and
   wherein the clamping force augmentation system includes a clamping force motor including the first clamping force gear and wherein the yoke includes the second clamping force gear.
5. The surgical system of embodiment 3, wherein the yoke includes a passageway having an opening defined on a proximal end of the yoke, and wherein the second clamping force gear is located within the passageway.
6. The surgical system of embodiment 5, wherein the first clamping force gear of the clamping force motor is configured to be received within the passageway of the yoke to engage with the second clamping force gear when the clamping force augmentation system is activated.
7. The surgical system of embodiment 6, further comprising a bias spring coupled to the yoke and the clamping force motor, the bias spring being configured to bias the first clamping force gear away from the second clamping force gear along the yoke axis.
8. The surgical system of embodiment 4, the second clamping force gear extends proximally away from a proximal end of the yoke.
9. The surgical system of embodiment 8, wherein the first clamping force gear is configured to axially mesh with the second clamping force gear when the clamping force augmentation system is engaged.
10. The surgical system of embodiment 4, wherein the engagement of the first clamping force gear with the second clamping force gear causes the yoke to move proximally along the yoke axis.
11. The surgical system of embodiment 1, wherein the controller is configured to deactivate the clamping force augmentation system in response to (i) the ultrasonic mode being activated or (ii) the position sensor indicating that the present state of the jaw assembly is not in the closed state.
12. A method for controlling operation of a surgical instrument, the method comprising:
   determining, by a controller of the surgical instrument, whether a jaw assembly of an end effector of the surgical instrument is in a closed state based on sensor data produced by a position sensor of a trigger assembly of the surgical instrument;
   determining, by the controller, whether a radio frequency (RF) mode of the surgical instrument has been activated; and
   activating, by the controller, a clamping force augmentation system of the surgical instrument to increase a clamping force of the jaw assembly of the end effector in response to a determination that (i) the jaw assembly is in the closed state and (ii) the RF mode has been activated.
13. The method of embodiment 1, wherein activating the clamping force augmentation system comprises engaging a first clamping force gear of the clamping force augmentation system and a second clamping force gear of the clamping force augmentation to increase the clamping force of the jaw assembly of the end effector.
14. The method of embodiment 13, wherein activating the clamping force augmentation comprises inserting the first clamping force gear into a passageway defined in a proximal end of a yoke of a trigger assembly of the surgical instrument to engage the second clamping force gear located within the passageway.
15. The method of embodiment 14, wherein engaging the first clamping force gear with the second clamping force gear comprises causing the yoke to move proximally along a yoke axis.
16. The method of embodiment 13, wherein activating the clamping force augmentation comprises engaging the first clamping force gear with the second clamping force gear that extends from a proximal end of a yoke of a trigger assembly of the surgical instrument.
17. The method of embodiment 16, wherein engaging the first clamping force gear with the second clamping force gear comprises axially meshing the first clamping force gear with the second clamping force gear.
18. The method of embodiment 12, further comprising deactivating, by the controller, the clamping force augmentation system in response to (i) determining, by the controller, that the RF mode has been deactivated or (ii) determining, by the controller, that the jaw assembly of the end effector is not in the closed state.
19. A surgical instrument comprising:
   an end effector having a jaw assembly movable between an open state and a closed state, wherein the jaw assembly includes an ultrasonic blade;
   a trigger assembly operable to move the jaw assembly of the end effector between the open state and the closed state, wherein the trigger assembly includes a position sensor configured to produce sensor data indicative of a present state of the jaw assembly of the end effector and a yoke configured to move along a yoke axis in response to operation of the trigger assembly to move the jaw assembly of the end effector between the open state and the closed state;
   a clamping force augmentation system controllable to increase a clamping force of the jaw assembly of the end effector, wherein the clamping force augmentation system comprises a first clamping force gear coupled to a clamping force motor and a second clamping force gear coupled to the yoke;
   a controller configured to control activation of a radio frequency (RF) mode of the surgical instrument, wherein the controller is configured to activate the clamping force augmentation system to cause engagement of the first clamping force gear with the second clamping force gear in response to a determination that (i) the RF mode is activated and (ii) the jaw assembly is in the closed state.
20. The surgical instrument of embodiment 19, wherein the yoke includes a passageway having an opening defined on a proximal end of the yoke, and wherein the second clamping force gear is located within the passageway,
   wherein to cause engagement of the first clamping force gear with the second clamping force gear comprises to receive the first clamping force gear within the passageway of the yoke.

While the disclosure has been illustrated and described in detail in the drawings and foregoing description, such an illustration and description is to be considered as illustrative and not restrictive in character, it being understood that only illustrative embodiments have been shown and described and that all changes and modifications that come within the spirit of the disclosure are desired to be protected.

There are a plurality of advantages of the present disclosure arising from the various features of the methods, apparatuses, and systems described herein. It will be noted that alternative embodiments of the methods, apparatuses, and systems of the present disclosure may not include all of the features described yet still benefit from at least some of the advantages of such features. Those of ordinary skill in the art may readily devise their own implementations of the methods, apparatuses, and systems that incorporate one or more of the features of the present invention and fall within the spirit and scope of the present disclosure as defined by the appended claims.

## Claims

1. A surgical instrument comprising:
an end effector having a jaw assembly movable between an open state and a closed state, wherein the jaw assembly includes an ultrasonic blade;
a trigger assembly operable to move the jaw assembly of the end effector between the open state and the closed state, wherein the trigger assembly includes a position sensor configured to produce sensor data indicative of a present state of the jaw assembly of the end effector;
a clamping force augmentation system controllable to increase a clamping force of the jaw assembly of the end effector;
a controller configured to control activation of an ultrasonic mode of the surgical system and a radio frequency (RF) mode of the surgical instrument, wherein the controller is configured to activate the clamping force augmentation system to increase the clamping force of the jaw assembly of the end effector in response to (i) the RF mode being activated and (ii) the position sensor indicating that the present state of the jaw assembly is in the closed state.

2. A surgical instrument comprising:
an end effector having a jaw assembly movable between an open state and a closed state, wherein the jaw assembly includes an ultrasonic blade;
a trigger assembly operable to move the jaw assembly of the end effector between the open state and the closed state, wherein the trigger assembly includes a position sensor configured to produce sensor data indicative of a present state of the jaw assembly of the end effector and a yoke configured to move along a yoke axis in response to operation of the trigger assembly to move the jaw assembly of the end effector between the open state and the closed state;
a clamping force augmentation system controllable to increase a clamping force of the jaw assembly of the end effector, wherein the clamping force augmentation system comprises a first clamping force gear coupled to a clamping force motor and a second clamping force gear coupled to the yoke;
a controller configured to control activation of a radio frequency (RF) mode of the surgical instrument, wherein the controller is configured to activate the clamping force augmentation system to cause engagement of the first clamping force gear with the second clamping force gear in response to a determination that (i) the RF mode is activated and (ii) the jaw assembly is in the closed state.

3. The surgical system of claim 1 or claim 2, wherein the trigger assembly includes a mode switch that is selectable to instruct the controller to activate the RF mode.

4. The surgical system of claim 1, wherein to activate the clamping force augmentation system comprises to engage a first clamping force gear of the clamping force augmentation system and a second clamping force gear of the clamping force augmentation system to increase the clamping force of the jaw assembly of the end effector.

5. The surgical system of claim 4, wherein the trigger assembly includes a yoke configured to move along a yoke axis in response to operation of the trigger assembly to move the jaw assembly of the end effector between the open state and the closed state, and
wherein the clamping force augmentation system includes a clamping force motor including the first clamping force gear and wherein the yoke includes the second clamping force gear.

6. The surgical system of claim 2 or claim 5, wherein the yoke includes a passageway having an opening defined on a proximal end of the yoke, and wherein the second clamping force gear is located within the passageway.

7. The surgical system of claim 6, wherein the first clamping force gear of the clamping force motor is configured to be received within the passageway of the yoke to engage with the second clamping force gear when the clamping force augmentation system is activated.

8. The surgical system of claim 7, further comprising a bias spring coupled to the yoke and the clamping force motor, the bias spring being configured to bias the first clamping force gear away from the second clamping force gear along the yoke axis.

9. The surgical system of claim 2 or any one of claims 5 to 8, the second clamping force gear extends proximally away from a proximal end of the yoke.

10. The surgical system of claim 9, wherein the first clamping force gear is configured to axially mesh with the second clamping force gear when the clamping force augmentation system is engaged.

11. The surgical system of claim 2 or any one of claims 5 to 10, wherein the engagement of the first clamping force gear with the second clamping force gear causes the yoke to move proximally along the yoke axis.

12. The surgical system of any preceding claim, wherein the controller is configured to deactivate the clamping force augmentation system in response to (i) the ultrasonic mode being activated or (ii) the position sensor indicating that the present state of the jaw assembly is not in the closed state.

13. A method for controlling operation of a surgical instrument, the method comprising:
determining, by a controller of the surgical instrument, whether a jaw assembly of an end effector of the surgical instrument is in a closed state based on sensor data produced by a position sensor of a trigger assembly of the surgical instrument;
determining, by the controller, whether a radio frequency (RF) mode of the surgical instrument has been activated; and
activating, by the controller, a clamping force augmentation system of the surgical instrument to increase a clamping force of the jaw assembly of the end effector in response to a determination that (i) the jaw assembly is in the closed state and (ii) the RF mode has been activated.

14. The method of claim 13, wherein activating the clamping force augmentation system comprises engaging a first clamping force gear of the clamping force augmentation system and a second clamping force gear of the clamping force augmentation to increase the clamping force of the jaw assembly of the end effector.

15. The method of claim 13 or claim 14, wherein activating the clamping force augmentation comprises inserting the first clamping force gear into a passageway defined in a proximal end of a yoke of a trigger assembly of the surgical instrument to engage the second clamping force gear located within the passageway.
